# EUROPEAN PATENT SPECIFICATION

(11) **EP 1 839 628 B1**
(45) Date of publication and mention of the grant of the patent: **10.12.2008**
(21) Application number: 07104110.7
(22) Date of filing: 14.03.2007
(51) Int. Cl.: A61F 9/00

(54) **System and method for user selectable release modality for a surgical cassette**
System und Verfahren für vom Benutzer wählbare Freigabemodalitäten einer Operationskassette
Système et procédé de modalité de libération paramétrable par l'utilisateur pour une cassette chirurgicale

(30) Priority: 31.03.2006 US 394896
(43) Date of publication of application: 03.10.2007
(73) Proprietor: Alcon, Inc., 6331 Hünenberg (CH)
(72) Inventor: Williams, David L., Newport Beach, CA 92663 (US)
(74) Representative: Hanna, Peter William Derek

(56) References cited:
- EP-A- 0 348 146
- US-A- 4 758 220
- US-A- 5 997 528

## Description

### TECHNICAL FIELD OF THE INVENTION

The present invention relates to surgical cassettes. More particularly, embodiments of the present invention relate to surgical cassettes used in ophthalmic surgical systems. Even more particularly, embodiments of the present invention relate to systems and methods for releasing surgical cassettes from surgical instrumentation.

### BACKGROUND OF THE INVENTION

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreoretinal surgery, and anterior segment procedures, such as cataract surgery. More recently, combined anterior and posterior segment procedures have been developed.

The surgical instrumentation used for ophthalmic surgery can be specialized for anterior segment procedures or posterior segment procedures or support both. In any case, the surgical instrumentation often requires the use of associated consumables such as surgical cassettes, fluid bags, tubing, hand piece tips and other consumables.

A surgical cassette can provide a variety of functions depending on the procedure and surgical instrumentation. For example, surgical cassettes for cataract surgeries (e.g., phacoemulsification procedures) help manage irrigation and aspiration flows into and out of a surgical site. Surgical cassettes can also provide support for fluid bags, a manifold for directing vacuum/pressure to surgical instrumentation, and other functionality.

Cassettes are generally coupled to the surgical instrumentation at a cassette receiving site. When a cassette is inserted into the cassette receiver, a clamp closes on the cassette to hold the cassette in place. Surgical cassettes are often released from the surgical instrumentation by pressing a release button or other mechanically electrical actuate that causes the surgical instrumentation to initiate the process of releasing the cassette. Unfortunately, if the cassette release button is accidentally activated, the cassette may release during a surgical procedure. Therefore, there is a need for a system and method that allows a user to select how a cassette should be released to prevent accidental releases.

Documents US 4,758,220, US 5,997,528 and EP 0 348 146 disclose surgical devices having a single release mode activated by a switch.

### SUMMARY OF THE INVENTION

Embodiments of the present invention provide a system and method for user selectable release modality of a surgical cassette. One embodiment of the present invention includes a method for user selectable cassette release modality. The method can comprise the steps of providing a user interface that allows a user to select a cassette release mode for a surgical cassette from a set of cassette release modes, determining that a release request is asserted, and releasing the surgical cassette from surgical instrumentation according to the selected cassette release mode. According to one embodiment, the release modes for the cassette can include an instant mode, a continuous mode and a delay mode.

Another embodiment of the present invention can include a computer program product comprising a set of computer instructions stored on a computer readable medium, said set of computer instructions comprising instructions executable by a processor to provide a user interface that allows a user to select a cassette release mode for a surgical cassette from a set of cassette release modes, determine that a release request is asserted, and issue one or more commands to release the surgical cassette according to the selected release mode.

Yet another embodiment of the present invention includes a surgical system comprising a display device, a cassette receiver to receive a surgical cassette, a cassette release button to request release of the surgical cassette from the cassette receiver and a controller coupled to (i.e., operable to communicate with) the display device and the cassette release button. The controller can be configured to provide a user interface to a user that allows the user to select a cassette release mode from a set of cassette release modes, receive a cassette release request signal when the release button is actuated and issue one or more commands to release the cassette from the cassette receiver according to the selected cassette release mode.

Embodiments of the present invention provide an advantage by allowing a user to select release modes that balance speed of release with prevention of accidental cassette release.

Embodiments of the present invention provide another advantage by allowing surgical equipment to have greater flexibility to support diverse users.

### BRIEF DESCRIPTION OF THE DRAWINGS

A more complete understanding of the present invention and the advantages thereof may be acquired by referring to the following description, taken in conjunction with the accompanying drawings in which like reference numbers indicate like features and wherein:
FIGURE 1 is a diagrammatic representation of one embodiment of a surgical console;
FIGURE 2 is a diagrammatic representation of one embodiment of a cassette receiver;
FIGURE 3 is a diagrammatic representation of one embodiment of a surgical cassette;
FIGURE 4 is a diagrammatic representation illustrating a cross-sectional view of one embodiment of a cassette in a cassette receiver;
FIGURE 5 is a diagrammatic representation of one embodiment of a controller for surgical instrumentation; and
FIGURE 6 is a flow chart illustrating one embodiment of a method for providing user selectable cassette release modality.

### DETAILED DESCRIPTION OF THE INVENTION

Preferred embodiments of the invention are illustrated in the FIGURES, like numerals being used to refer to like and corresponding parts of the various drawings.

Embodiments of the present invention provide a system and method for allowing a user to select the release modality of a surgical cassette. According to one embodiment of the present invention, a user can be allowed to choose one of multiple modes of operation for releasing a surgical cassette. For example, the user can choose a first mode of operation in which the cassette release process begins and proceeds to completion when a user presses a release button regardless of how long the user holds the release button. In a second mode of operation, the user must continue to hold the button for the entire release process. If the button is not continuously held, the surgical instrumentation will stop the release command, or stop the release command and then regrip the cassette. In a third mode of operation, the user must hold the release button for a predefined delay period. If the user holds the button for the predefined delay period, the surgical instrumentation will commence cassette release and proceed to finish.

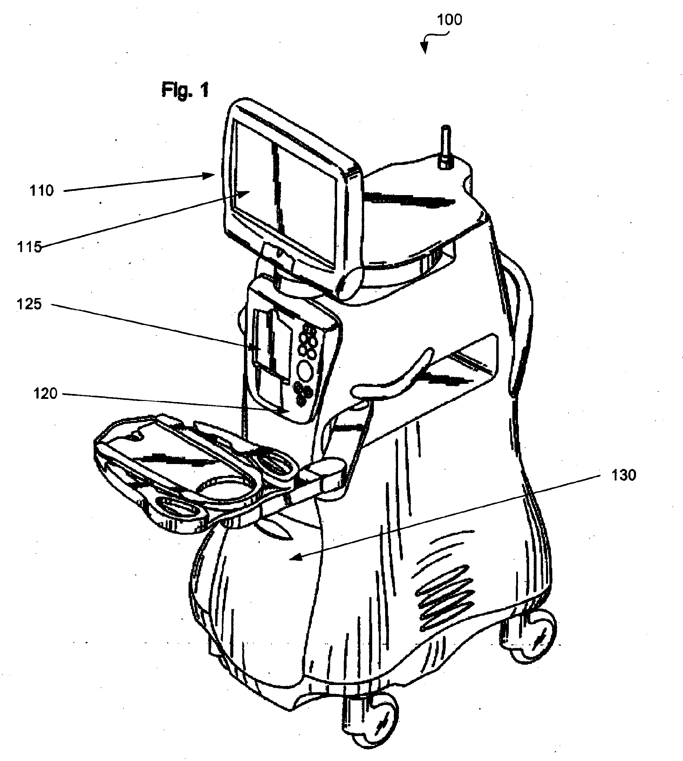

FIGURE 1 is a diagrammatic representation of one embodiment of an ophthalmic surgical console 100. Surgical console 100 can include a swivel monitor 110 that has touch screen 115. Swivel monitor 110 can be positioned in a variety of orientations for whomever needs to see touch screen 115. Swivel monitor 110 can swing from side to side, as well as rotate and tilt. Touch screen 115 provides a graphical user interface ("GUI") that allows a user to interact with console 100.

Surgical console 100 also includes a connection panel 120 used to connect various tools and consumables to surgical console 100. Connection panel 120 can include, for example, a coagulation connector, balanced salt solution receiver, connectors for various hand pieces and a fluid management system ("FMS") or cassette receiver 125. Surgical console 100 can also include a variety of user friendly features, such as a foot pedal control (e.g., stored behind panel 130) and other features.

In operation, a cassette (not shown) can be placed in cassette receiver 125. Clamps in surgical console 100 clamp the cassette in place to minimize movement of the cassette during use. The clamps can clamp the top and bottom of the cassette, the sides of the cassette or otherwise clamp the cassette.

Surgical console 100 is provided by way of example and embodiments of the present invention can be implemented with a variety of surgical systems. Example surgical systems in which cassettes according to various embodiments of the present invention can be used include, for example, the Series 2000® Legacy® cataract surgical system, the Accurus® 400VS surgical system, and the Infiniti^{™} Vision System surgical system, all available from Alcon Laboratories Inc. of Fort Worth, Texas. Additionally, embodiments of the present invention can be used with a variety of surgical cassettes, examples of which are described in U.S. Pub. Nos. 2005/0186098 (Application No. 11/114,289 to Davis et al.), 2004/0253129 (Application No. 10/891,642 to Sorensen et al.), 2005/0065462 (Application No. 10/979,433 to Nazarifar et al.), 2003/0225363 (Application No. 10/156,175 to Gordon et al.), 2001/0016711 (Application No. 09/846,724 to Sorensen et al.) and United States Patent Nos. 6,293,926 to Sorensen et al., 4,493,695 to Cook, 4,627,833 to Cook, 4,395,258 to Wang et al, 4,713,051 to Steppe, et al., 4,798,850 to DeMeo, et al., 4,758,238 to Sundblom et al, 4,790,816 to Sundblom et al., 6,267,956 to Beuchat, 6,364,342 to Beuchat, 6,036,458 to Cole et al and 6,059,544 to Jung et al., Embodiments of the present invention can be implemented for other suitable surgical systems and cassettes as would be understood by one of ordinary skill in the art.

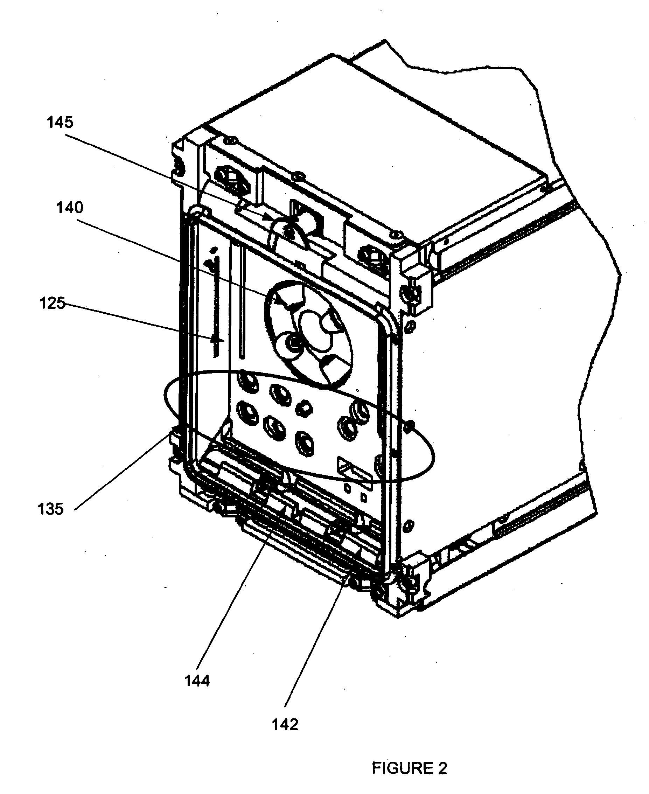

FIGURE 2 is a diagrammatic representation of one embodiment of cassette receiver 125 without a cassette. Cassette receiver 125 can have various input and output ports (indicated generally at 135) to receive fluids (i.e., liquids and gasses) from the surgical cassette. Cassette receiver 125 can further include an opening to allow peristaltic pump rollers 140 to contact the surgical cassette during operation. One embodiment of a peristaltic pump and complimentary cassette is described in United States Patent Application No. 6,293,926 to Sorensen,

The surgical cassette, in the embodiment of FIGURE 2, is held in place by a clamp having a bottom rail 142 and a top rail (not shown). Each rail can have clamping fingers (e.g., clamp finger 144) that contact the cassette in corresponding clamping zones. One embodiment of a surgical cassette clamp is described in United States Patent Pub. No. 2003/0202894 (Application No. 10/132,797). A release button 145 is pressed to initiate release of the cassette from the clamp. Depending on the surgical console 100, the cassette release process can include several steps, including venting of pressure or fluids, disengaging the clamps or other steps. The configuration of FIGURE 2 is provided by way of example. The form factor of cassette receiver 125, placement and number of input/output ports and other features of cassette receiver 125 can depend on the surgical console 100, on the surgical procedure being performed or on other factors.

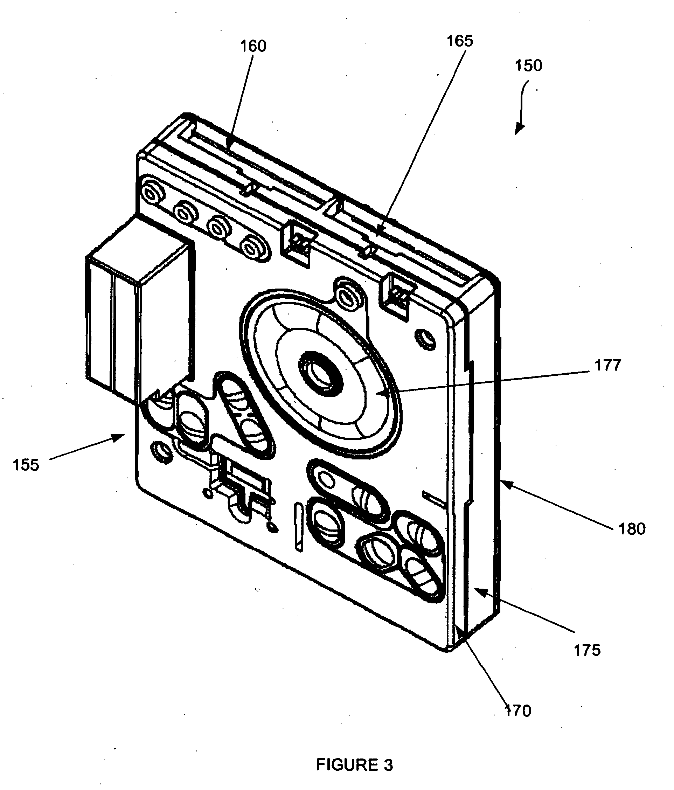

FIGURE 3 is a diagrammatic representation of one embodiment of a surgical cassette 150. Cassette 150 can provide a closed system fluidic device that can be discarded following a surgical procedure. Cassette 150 can include a cassette body 155 and clamp receiving portions (e.g., indicated generally at clamping zones 160 and 165) projecting from the cassette body 155. In the embodiment shown, cassette 150 is formed from three primary sections: an inner or surgical console interface section 170 that faces the surgical console when cassette 150 is inserted into surgical console 100, a middle section 175 and a back plate 180. The various sections of cassette 150 can be coupled together via a press fit, interlocking tabs, chemical bonding, thermal bonding, mechanical fasteners or other attachment mechanism known in the art.

Surgical console interface section 170 can provide an interface for fluid flow channels (e.g., flow channel 177 for the peristaltic pump provided by an elastomeric pump membrane), valves (e.g., irrigation/aspiration valves), pressure sensors and other features to manage fluid flow. Cassette 150 can also attach to a fluid bag (not shown) to collect fluids during a procedure.

In operation, cassette 150 is held in place in cassette receiver 125 by clamp rails that contact cassette 150 in the clamping zones. For example, the upper clamp rail will contact cassette 150 in clamping zone 160 and clamping zone 165 while the bottom clamp rail (e.g., bottom clamp rail 142) will contact cassette 150 at similar bottom clamping zones.

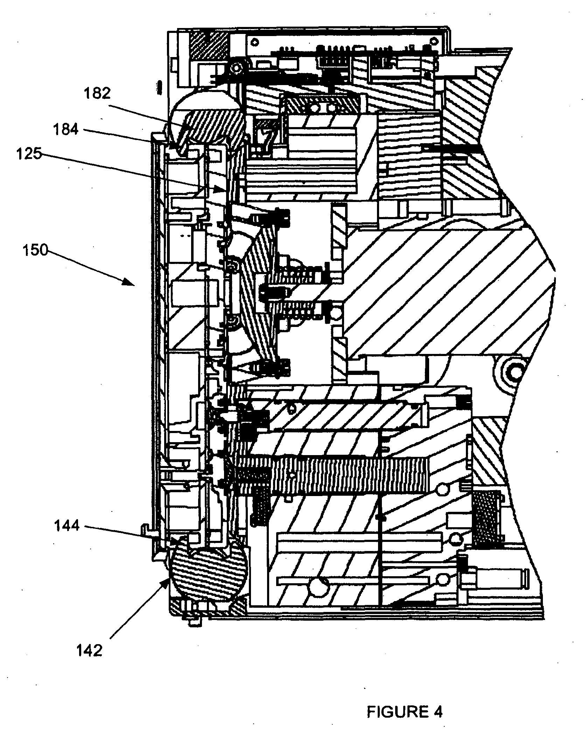

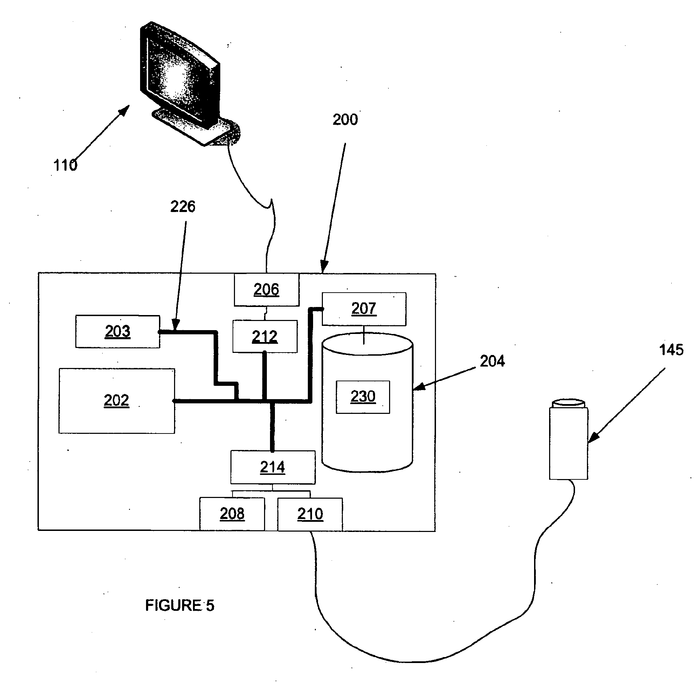

FIGURE 4 is a cross-section of one embodiment of cassette 150 inserted into cassette receiver 125. Cassette 150 is held in place by a clamp. In the embodiment of FIGURE 5, the clamp includes lower clamp rail 142 and upper clamp rail 182, though in other embodiments the clamp can contact cassette 150 in other areas. When cassette 150 is initially inserted, clamp rails 142/182 rotate so that clamping fingers (e.g., clamp finger 144 and clamp finger 184) contact cassette 150 in the clamping zones. Rotation can be imparted to the clamp rails 142/182 from the force of insertion, by an air cylinder, by a motor or any combination of these. To release the cassette, the clamp rails 142/182 rotate in the opposite direction. When inserted, surgical console interface section 155 can contact surgical console 100 such that, for example, peristaltic pump rollers 140 can squeeze flow channel 177.

FIGURE 5 is a diagrammatic representation of a surgical instrumentation controller 200 ("controller 200"). Controller 200 can be onboard or connected to surgical instrumentation such as surgical console 100. Controller 200 can include a processor 202, such as an Intel Pentium 4® based processor (Intel and Pentium are trademarks of Intel Corporation of Santa Clara, California), a primary memory 203 (e.g., RAM, ROM, Flash Memory, EEPROM or other computer readable medium known in the art) and a secondary memory 204 (e.g., a hard drive, disk drive, optical drive or other computer readable medium known in the art). A memory controller 207 can control access to secondary memory 204. Controller 200 can include I/O interfaces, such as video interface 206 and I/O interfaces 208 and 210 to connect to other devices. Controller 200 can receive data from a release indicator (e.g., release button 145) via I/O interface 210 and send commands to the system via I/O interface 208. A video controller 212 can control interactions over the video interface 206 and an I/O controller 214 can control interactions over I/O interfaces 208 and 210. Controller 200 can include a variety of input devices. Various components of controller 200 can be connected by a bus 226.

Secondary memory 204 can store a variety of computer instructions that include, for example, an operating system such as a Windows® operating system (Windows is a trademark of Redmond, Washington based Microsoft Corporation) and applications that run on the operating system, along with a variety of data. More particularly, secondary memory 204 can store a software program 230 that controls the release mode of the surgical cassette. During execution by processor 202, portions of program 230 can be stored in secondary memory 204 and/or primary memory 203.

In operation, program 230 can be executable by processor 202 to provide a GUI to the user (e.g., through monitor 110) that allows the user to select the release mode for surgical cassette 150. The release mode can be based on the actuation of release button 145 or other indication of a release request. According to one embodiment, the user can select a latching release mode, a continuous release mode or a delayed release mode. Each release mode is different in the amount of time release button 145 must be pressed (or that the user must otherwise indicate that a release is requested), or the way in which the software reacts in order to release the cassette. According to one embodiment of the present invention, the selected mode can become the default mode until a user changes the selected mode.

Under a first mode of operation, when controller 200 receives a release request (e.g., by detecting actuation of release button 145 or other indication of a release request) controller 200 will commence the cassette release process and proceed to finish (i.e., the point where the user can safely remove the cassette from the surgical instrumentation). This mode of operation is fast and convenient for a user as the user can push release button 145 once and walk away until the release process is finished.

Under a second mode of operation, when controller 200 receives a release request, controller 200 can initiate the release process (e.g., by sending commands or control signals to the clamping mechanism or other components of the surgical instrumentation). Controller 200 can continue to determine if a release request is being asserted by the user throughout the release process and, if not, controller 200 can halt the release process. Using the example in which the user presses release button 145 to request a release, controller 200, according to the second mode of operation, can continually sample the signal from release button 145 (i.e., within the hardware or software sampling rate of controller 200) to determine if the user continuously actuated the release button. If, before the release process finishes, controller 200 detects that the user is no longer pressing release button 145, controller 200 can halt the cassette release process. While this mode of operation may not be as convenient for the user as it does not allow the user to walk away during the release process, it eliminates or at least substantially reduces accidental button actuation causing release of the cassette.

A third, or delay, mode of operation is similar to the first mode of operation, but a time delay is included. The time delay can be hard-coded, pre-programmed by the supplier of the surgical instrumentation or user-programmable. Under the third mode of operation, when controller 200 detects a release request (e.g., by detecting actuation of release button 145), controller 200 will continuously determine if the release request is being asserted by the user for a delay period. If controller 200 determines that the release request is asserted by the user for the entire delay period, controller 200 can commence the release process and proceed to finish. Conversely, if controller 200 determines that the user ceases asserting the release request during the delay period (e.g., by no longer pressing release button 145), controller 200 will not commence the release process. The time delay introduced in reading of the release request helps prevent accidental button actuation from accidentally releasing the cassette. By requiring that the release request be asserted for a predetermined period of time, the possible event of an accidental transient actuation of the release button is filtered out. This mode of operation can be almost as fast as the first mode of operation, while affording much of the protection of the second mode of operation.

It should be noted that the modes of operation are provided by way of example, but not limitation and other modes of operation can be implemented. According to one embodiment, the differences between the three modes of operation can be implemented through program 230. Using the example of a cassette release button to request a cassette release, the first mode of operation can use a software latch on the button actuation signal to start and complete the cassette release. The second mode of operation uses a continuous button signal to complete the cassette release from start to completion. The third mode of operation uses a time delay in program 230 such that the button signal is required to have a minimum time duration before a latch is used to start and complete the release.

Controller 200 of FIGURE 5 is provided by way of example only and it should be understood that embodiments of the present invention can be implemented as a set of computer instructions stored on a computer readable medium in a variety of computing devices. Program 230 can be executable to receive and store data over a network and can include instructions that are stored at a number of different locations and are executed in a distributed manner. While shown as a stand alone program in FIGURE 5, it should be noted that program 230 can be a module of a larger program, can comprise separate programs operable to communicate data to each other or can be implemented according to any suitable programming architecture and language.

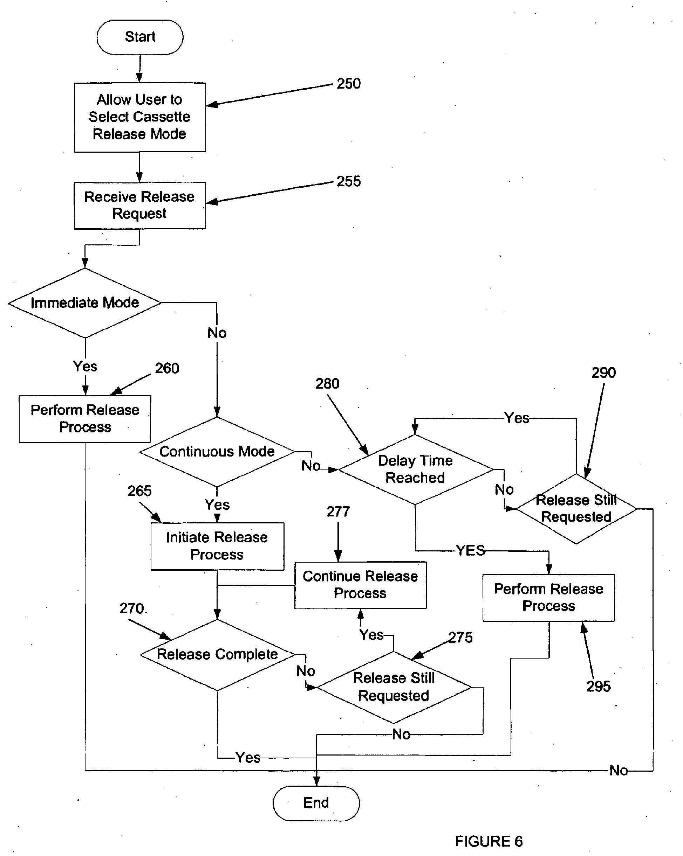

FIGURE 6 is a flow chart illustrating one embodiment of a method for user selectable release modality. The method of FIGURE 6 can be implemented as a set of computer executable instructions stored on a computer readable medium at, for example, surgical console 100. The surgical console, at step 250, can present the user with a user interface (e.g., a GUI) that allows the user to select a cassette release mode. The modes can include, for example, an immediate mode, a continuous mode and a delay mode. Additionally, the user interface can allow the user to provide parameters for the selected mode. For example, the user can optionally be given the opportunity to select the delay time for the delay mode. As another example, the user can select whether the release must be triggered by one of release button 145 or a button in the GUI, or whether the release can be triggered by either or some other mechanism.

At step 255, surgical console 100 can receive a release request. The release request can be asserted, for example, by the user pressing a cassette release button, providing an input through the graphical user interface or otherwise requesting release of the surgical cassette. If the user selected the immediate mode, surgical console 100 can commence the release process and proceed to finish (step 260), at which time the user can remove the cassette.

If the user selected continuous mode, surgical console 100 can initiate the release process (step 265). At step 270, surgical console can determine if the release process has completed and, if not, determine if the release request is still asserted by the user (step 275). This can be done, for example, by determining if the release button is still pressed or otherwise determining that the user is continuing to request the release. If the release request is still asserted, console 100 can continue the release process (step 277) and return to step 270. Steps 270, 275 and 277 can be repeated until the release process is completed (e.g., as determined at step 270) or the release request is no longer asserted (e.g., as determined at step 275). If surgical console 100 determines that the release request is no longer asserted prior to the release process completing, surgical console 100 can terminate the release process prior to completion, or terminate the release prior to completion and then regrip the cassette.

If the user selected the delay mode, surgical console 100, at step 280, can determine if the delay time has been reached. If the delay time has not been reached, surgical console 100, at step 290, can determine if the release request is still asserted. Again this can be done by sampling the release button signal to determine if the release button is still actuated. If surgical console 100 determines that the release request is asserted for the delay time, surgical console 100 can commence the release process and proceed to finish (step 295). If, however, surgical console 100 determines that the release request is no longer asserted during the delay time, surgical console 100 will not commence the release process.

The steps of FIGURE 6 can be repeated as needed or desired. Moreover, FIGURE 6 is provided by way of example and embodiments of the present invention can implement the steps in different orders or provide more or fewer modes of operation.

Embodiments of the present invention can be used in a variety of surgical systems, particularly ophthalmic surgical systems, and provide increased functionality and ease of use. Users are able to balance their preferences on speed versus accidental release. For example, cataract users may value cassette release speed more than preventing accidental releases, since cataract surgeries are generally short duration procedures in which setup and teardown time is a large percentage of the total procedure time. An accidental cassette release during a cataract surgery would generally not be critical since the interruption of irrigation or aspiration functions would not typically cause a patient harm. Vitreoretinal users, on the other hand, may prefer preventing accidental releases more than cassette release speed, since these surgeries are typically long duration procedures in which the setup and teardown time is a small percentage of the total case time. Since an accidental release of the cassette during such a procedure would interrupt the infusion function and cause a possible recalibration of the system and a potential and unintended change in intraocular pressure, an accidental release is undesirable. By allowing the user to select a preferred release mode, surgical instrumentation can have greater flexibility to support diverse users.

While the present invention has been described with reference to particular embodiments, it should be understood that the embodiments are illustrative and that the scope of the invention is not limited to these embodiments. Many variations, modifications, additions and improvements to the embodiments described above are possible. It is contemplated that these variations, modifications, additions and improvements fall within the scope of the invention as detailed in the following claims.

## Claims

1. A computer-implemented method for controlling the surgical cassette (150) release mechanism (142,144,182,184) in a surgical system (100), comprising the steps of;
receiving (255) user input entered via a user interface (115), the user interface allowing a user to select (250) a cassette release mode for the surgical cassette from a set of cassette release modes;
determining that a cassette release request is asserted; and
issuing one or more commands (260,265,295) to cause the surgical cassette to be released from a cassette receiver (125) according to the selected cassette release mode.

2. The method of claim 1, wherein issuing one or more commands to cause the surgical cassette to be released from the surgical console according to the selected cassette release mode, comprises;
if the selected cassette release mode is an immediate mode,
initiating (260) and completing a cassette release process based on determining that the release request is asserted.

3. The method of claim 1, wherein issuing one or more commands to cause the surgical cassette to be released from the surgical console according to the selected cassette release mode, comprises;
if the selected cassette release mode is a continuous mode,
initiating (265) and completing a cassette release process based on determining that the release request is asserted,
subsequently determining (275) if the release request is still asserted prior to completion of the cassette release process,
if the release request is still not asserted and the cassette release process has not completed, terminating the cassette release process.

4. The method of claim 3, wherein subsequently determining (275) if the release request is still asserted prior to completion of the cassette release process further comprises continually determining whether the release request is still asserted until the release process is completed (270) or terminated.

5. The method of claim 3, wherein terminating the cassette release process further comprises reengaging the cassette.

6. The method of claim 1, wherein issuing one or more commands to cause the surgical cassette to be released from the surgical console according to the selected cassette release mode, comprises;
if the selected cassette release mode is a delay mode,
determining if the release request is asserted for a delay time (280); and
if so, initiating (295) the cassette release process.

7. The method of claim 6, further comprising continuing the cassette release process (277) until the cassette release process completes (270).

8. The method of claim 6, wherein the user interface (115) further allows the user to specify the delay time.

9. The method of any one of claims 1 to 8, wherein the step of determining that a cassette release request is asserted comprises determining whether a release command is asserted through input at the user interface (115) or by a signal received on actuation of a release button (145), and wherein the user can select how the release command is to be asserted.

10. A computer program (230), which when executing on a computer processor (202) performs the method of any one of claims 1 to 9.

11. The computer program (230) of claim 10, when stored on a computer readable medium.

12. A surgical system (100) comprising:
a display device (110);
a cassette receiver (125) to receive a surgical cassette (150);
a cassette release button (145) to request release of the surgical cassette from the cassette receiver;
a controller (200) coupled to the display device and the cassette release button, the controller configured to:
receive a cassette release request signal when the release button is actuated; and **characterized in that** the controller in further configured to display a user interface (115) to a user that allows the user to select a cassette release mode from a set of cassette release modes, and issue one or more commands to cause the surgical cassette to be released from the cassette receiver according to the selected cassette release mode on receiving the cassette release signal.

13. The surgical system of claim 12, wherein the set of cassette release modes comprises at least an immediate mode, a delay mode and a continuous mode.

14. The surgical system of claim 12, wherein the user interface (115) is adapted to allow the user to specify a delay time if the user selects the delay mode.

15. The surgical system of claim 13, wherein the controller (200) is further configured to:
if the immediate mode is selected,
initiate (260) and complete a cassette release process based on determining that the cassette release button (145) is pressed;
if the continuous mode is selected,
initiate (265) the cassette release process based on determining that the cassette release button is pressed;
continuously determine if the cassette release button is pressed;
if it is determined that the cassette release button is no longer pressed and the cassette release process has not completed, terminate the cassette release process; and
if the delay mode is selected,
determine (280) if the cassette release button is pressed for a delay time; and
if so, initiate (295) the cassette release process.

16. The surgical system of any one of claims 12 to 15, wherein the controller (200) is configured to determine whether a release command is asserted through input at the user interface (115) or by a signal received on actuation of a release button (145), and wherein the user can select how the release command is to be asserted.

## Patentansprüche

1. Computerimplementiertes Verfahren zum Steuern eines Freigabe-Mechanismus (142, 144, 182, 184) von einer Operations-Kassette (150) in einem Operationssystem (100), welches die Schritte enthält:
Empfangen (255) von einer Benutzereingabe, welche über eine Benutzer-Schnittstelle (115) eingegeben wird, wobei es die Benutzer-Schnittstelle einem Benutzer erlaubt, einen Kassetten-Freigabemodus für die Operations-Kassette aus einem Satz von Kassetten-Freigabemodi auszuwählen (250);
Bestimmen, dass eine Kassetten-Freigabeanforderung erklärt ist; und
Ausgeben von einem oder mehreren Befehlen (260, 265, 295), um zu bewirken, dass die Operations-Kassette aus einer Kassetten-Aufnahme (125) gemäß dem ausgewählten Kassetten-Freigabemodus freigegeben wird.

2. Verfahren nach Anspruch 1, bei welchem die Ausgabe von einem oder mehreren Befehlen, um zu bewirken, dass die Operations-Kassette aus der Operations-Konsole gemäß dem ausgewählten Kassetten-Freigabemodus freigegeben wird, enthält:
wenn der ausgewählte Kassetten-Freigabemodus in einem direkten Modus ist, Einleiten (260) und Vollenden eines Kassetten-Freigabeprozesses, basierend auf der Bestimmung, dass die Freigabe-Anforderung erklärt ist.

3. Verfahren nach Anspruch 1, bei welchem die Ausgabe von einem oder mehreren Befehlen, um zu bewirken, dass die Operations-Kassette aus der Operations-Konsole gemäß dem ausgewählten Kassetten-Freigabemodus freigegeben wird, enthält:
wenn der ausgewählte Kassetten-Freigabemodus in einem kontinuierlichen Modus ist,
Einleiten (265) und Vollenden eines Kassetten-Freigabeprozesses, basierend auf der Bestimmung, dass die Freigabe-Anforderung erklärt ist,
nachfolgendes Bestimmen (275), ob die Freigabe-Anforderung immer noch vor der Vollendung des Kassetten-Freigabeprozesses erklärt ist,
Beenden, wenn die Freigabe-Anforderung immer noch nicht erklärt ist und der Kassetten-Freigabeprozess nicht vollendet ist, des Kassetten-Freigabeprozesses.

4. Verfahren nach Anspruch 3, bei welchem ein nachfolgendes Bestimmen (275), ob die Freigabe-Anforderung immer noch vor der Vollendung von dem Kassetten-Freigabeprozess erklärt ist, ferner ein fortwährendes Bestimmen enthält, ob die Freigabe-Anforderung immer noch erklärt ist, bis der Freigabeprozess vollendet (270) oder abgeschlossen ist.

5. Verfahren nach Anspruch 3, bei welchem das Abschließen des Kassetten-Freigabeprozesses ferner eine Wiederineingriffnahme von der Kassette enthält.

6. Verfahren nach Anspruch 1, bei welchem die Ausgabe von einem oder mehreren Befehlen, um zu bewirken, dass die Operations-Kassette aus der Operations-Konsole gemäß dem ausgewählten Kassetten-Freigabemodus freizugeben ist, enthält:
wenn der ausgewählte Kassetten-Freigabemodus in einem Verzögerungsmodus ist,
Bestimmen, ob die Freigabe-Anforderung für eine Verzögerungszeit erklärt ist (280); und
Einleiten (295), wenn dies der Fall ist, des Kassetten-Freigabeprozesses.

7. Verfahren nach Anspruch 6, welches ferner ein Fortsetzen des Kassetten-Freigabeprozesses (277) enthält; bis der Kassetten-Freigabeprozess vollendet ist (270).

8. Verfahren nach Anspruch 6, bei welchem es die Benutzer-Schnittstelle (115) dem Benutzer ferner ermöglicht, die Verzögerungszeit zu spezifizieren.

9. Verfahren nach einem der Ansprüche 1 bis 8, bei welchem der Schritt des Bestimmens, dass eine Kassetten-Freigabeanforderung erklärt ist, ein Bestimmen enthält, ob ein Freigabebefehl über eine Eingabe an der Benutzerschnittstelle (115) oder durch ein Signal, welches bei einer Betätigung von einer Freigabetaste (145) empfangen wird, erklärt wird, und wobei der Benutzer auswählen kann, wie der Freigabebefehl zu erklären ist.

10. Computerprogramm (230), welches bei einer Ausführung auf einem Computerprozessor (202) das Verfahren nach einem der Ansprüche 1 bis 9 durchführt.

11. Computerprogramm (230) nach Anspruch 10, gespeichert auf einem computerlesbaren Medium.

12. Operationssystem (100), welches enthält:
eine Anzeigevorrichtung (110);
eine Kassetten-Aufnahme (125) zur Aufnahme von einer Operations-Kassette (150);
eine Kassetten-Freigabetaste (145) zur Anforderung von einer Freigabe der Operations-Kassette aus der Kassetten-Aufnahme;
eine Steuerung (200), welche an die Anzeigevorrichtung und die Kassetten-Freigabetaste gekoppelt ist, wobei die Steuerung dazu ausgelegt ist, um:
ein Kassetten-Freigabe-Anforderungssignal zu empfangen, wenn die Freigabetaste betätigt ist; und
**dadurch gekennzeichnet, dass** die Steuerung ferner dazu ausgelegt ist, um:
einem Benutzer eine Benutzer-Schnittstelle (115) anzuzeigen, welche es dem Benutzer erlaubt, einen Kassetten-Freigabemodus aus einem Satz von Kassetten-Freigabemodi auszuwählen; und
einen oder mehrere Befehle auszugeben, um zu bewirken, dass die Operations-Kassette aus der Kassetten-Aufnahme gemäß dem ausgewählten Kassetten-Freigabemodus beim Empfangen des Kassetten-Freigabesignals freigegeben wird.

13. Operationssystem nach Anspruch 12, bei welchem der Satz von Kassetten-Freigabemodi zumindest einen direkten Modus, einen Verzögerungsmodus und einen kontinuierlichen Modus enthält.

14. Operationssystem nach Anspruch 12, bei welchem die Benutzer-Schnittstelle (115) dazu ausgelegt ist, es dem Benutzer zu erlauben, eine Verzögerungszeit zu spezifizieren, wenn der Benutzer den Verzögerungsmodus auswählt.

15. Operationssystem nach Anspruch 13, bei welchem die Steuerung (200) ferner dazu ausgelegt ist, um:
wenn der direkte Modus ausgewählt ist,
einen Kassetten-Freigabeprozesses basierend auf der Bestimmung, dass die Kassetten-Freigabetaste (145) gedrückt ist, einzuleiten und zu bestimmen;
wenn der kontinuierliche Modus ausgewählt ist,
den Kassetten-Freigabeprozesses basierend auf der Bestimmung, dass die Kassetten-Freigabetaste gedrückt ist, einzuleiten (265);
kontinuierlich zu Bestimmen, ob die Kassetten-Freigabetaste gedrückt ist;
wenn bestimmt ist, dass die Kassetten-Freigabetaste nicht länger gedrückt ist und der Kassetten-Freigabeprozess nicht vollendet wurde, den Kassetten-Freigabeprozess abzuschließen; und
wenn der Verzögerungsmodus ausgewählt ist,
zu Bestimmen (280), ob die Kassetten-Freigabetaste für eine Verzögerungszeit gedrückt ist; und
wenn dies der Fall ist, den Kassetten-Freigabeprozess einzuleiten (295).

16. Operationssystem nach einem der Ansprüche 12 bis 15, bei welchem die Steuerung (200) dazu ausgelegt ist, um zu bestimmen, ob ein Freigabebefehl über eine Eingabe an der Benutzerschnittstelle (115) oder durch ein Signal, welches bei einer Betätigung von einer Freigabetaste (145) empfangen ist, erklärt ist, und wobei der Benutzer auswählen kann, wie der Freigabebefehl zu erklären ist.

## Revendications

1. Procédé implémenté par ordinateur pour commander le mécanisme de libération (142, 144, 182, 184) d'une cassette chirurgicale (150) dans un système chirurgical (100), comprenant les étapes consistant à :
recevoir (255) une entrée d'utilisateur entrée via une interface utilisateur (115), l'interface utilisateur permettant à un utilisateur de sélectionner (250) un mode de libération de cassette pour la cassette chirurgicale à partir d'un ensemble de modes de libération de cassette,
déterminer qu'une requête de libération de cassette est demandée, et
délivrer une ou plusieurs instructions (260, 265, 295) pour provoquer la libération de la cassette chirurgicale depuis un récepteur de cassette (125) conformément au mode de libération de cassette sélectionné.

2. Procédé selon la revendication 1, dans lequel la délivrance d'une ou plusieurs instructions pour provoquer la libération de la cassette chirurgicale de la console chirurgicale conformément au mode de libération de cassette sélectionné comporte :
si le mode de libération de cassette sélectionné est un mode immédiat,
le déclenchement (160) et l'achèvement d'un processus de libération de cassette sur la base de la détermination que la requête de libération est demandée.

3. Procédé selon la revendication 1, dans lequel la délivrance d'une ou plusieurs instructions pour provoquer la libération de la cassette chirurgicale de la console chirurgicale conformément au mode de libération de cassette sélectionné comporte :
si le mode de libération de cassette sélectionné est un mode continu,
le déclenchement (265) et l'achèvement d'un processus de libération de cassette sur la base de la détermination que la requête de libération est demandée,
la détermination consécutive (275) du fait que la requête de libération est toujours demandée avant achèvement du processus de libération de cassette,
si la requête de libération n'est toujours pas demandée, et le processus de libération de cassette n'a pas été achevé, l'achèvement du processus de libération de cassette.

4. Procédé selon la revendication 3, dans lequel la suite de la détermination (275), si la requête de libération est encore demandée avant achèvement du processus de libération de cassette, comporte en outre la détermination en continu de savoir si la requête de libération est encore demandée ou pas jusqu'à ce que le processus de libération soit achevé (270) ou terminé.

5. Procédé selon la revendication 3, dans lequel la fin du processus de libération de cassette comporte en outre le réengagement de la cassette.

6. Procédé selon la revendication 1, dans lequel la délivrance d'une ou plusieurs instructions pour provoquer la libération de la cassette chirurgicale de la console chirurgicale conformément au mode de libération de cassette sélectionné comprend :
si le mode de libération de cassette sélectionné est un mode retardé,
la détermination du fait que la requête de libération est demandée pendant un temps de retard (280), et
si c'est le cas, le déclenchement (295) du processus de libération de cassette.

7. Procédé selon la revendication 6, comprenant en outre la poursuite du processus de libération de cassette (67) jusqu'à ce que le processus de libération de cassette (270) soit terminé.

8. Procédé selon la revendication 6, dans lequel l'interface utilisateur (115) permet en outre à l'utilisateur de spécifier le temps de retard.

9. Procédé selon l'une quelconque des revendications 1 à 8, dans lequel l'étape consistant à déterminer qu'une requête de libération de cassette est demandée comprend la détermination du fait qu'une instruction de libération est demandée par une entrée au niveau de l'interface utilisateur (115) ou par un signal reçu lors de l'actionnement d'un bouton de libération (145), et dans lequel l'utilisateur peut sélectionner la manière dont l'instruction de libération doit être demandée.

10. Programme informatique (230), qui lorsqu'il est exécuté sur un processeur informatique (202), met en oeuvre le procédé selon l'une quelconque des revendications 1 à 9.

11. Programme informatique (230), lorsque mémorisé sur un support pouvant être lu par ordinateur.

12. Système chirurgical (100), comportant :
un dispositif d'affichage (110),
un récepteur de cassette (125) destiné à recevoir une cassette chirurgicale (150),
un bouton de libération de cassette (145) pour demander une libération de cassette chirurgicale à partir du récepteur de cassette,
un contrôleur (200) couplé dispositif d'affichage et au bouton de libération de cassette, le contrôleur étant configuré pour :
recevoir un signal de requête de libération de cassette lorsque le bouton de libération est actionné, et **caractérisé en ce que** le contrôleur est en outre configuré pour afficher une interface utilisateur (115) à un utilisateur, qui permet à l'utilisateur de sélectionner un mode de libération de cassette à partir d'un ensemble de modes de libération de cassette, et délivre une ou plusieurs instructions pour provoquer la libération de la cassette chirurgicale du récepteur de cassette conformément au mode de libération de cassette sélectionné à la réception du signal de libération de cassette.

13. Système chirurgical selon la revendication 12, dans lequel l'ensemble de modes de libération de cassette comporte au moins un mode immédiat, un mode retardé et un mode continu.

14. Système chirurgical selon la revendication 12, dans lequel l'interface utilisateur (115) est adaptée pour permettre à l'utilisateur de spécifier un temps de retard si l'utilisateur sélectionne le mode retardé.

15. Système chirurgical selon la revendication 13, dans lequel le contrôleur (200) est en outre configuré pour :
si le mode immédiat est sélectionné,
déclencher (260) et achever un processus de libération de cassette sur la base de la détermination que le bouton de libération de cassette (145) est enfoncé,
si le mode continu est sélectionné,
déclencher (265) le processus de libération de cassette sur la base de la détermination que le bouton de libération de cassette est enfoncé,
déterminer en continu si le bouton de libération de cassette est enfoncé,
s'il est déterminé que le bouton de libération de cassette n'est plus enfoncé, et le processus de libération de cassette n'a pas été achevé, terminer le processus de libération de cassette, et
si le mode retardé est sélectionné, déterminer (180) si le bouton de libération de cassette est enfoncé pendant un temps de retard, et
si c'est le cas, déclencher (295) le processus de libération de cassette.

16. Système chirurgical selon l'une quelconque des revendications 12 à 15, dans lequel le contrôleur (200) est configuré pour déterminer si une instruction de libération est demandée par une entrée au niveau de l'interface utilisateur (115) ou par un signal reçu lors de l'actionnement d'un bouton de libération (145), et dans lequel l'utilisateur peut sélectionner la manière dont l'instruction de libération doit être demandée.
